# EUROPÄISCHE PATENTSCHRIFT

(11) **EP 3 766 957 B1**
(45) Veröffentlichungstag und Bekanntmachung des Hinweises auf die Patenterteilung: **11.03.2026**
(21) Anmeldenummer: 19187054.2
(22) Anmeldetag: 18.07.2019
(51) Int. Cl.: C12M 1/12, A61L 27/60, B33Y 10/00, C12M 3/00

(54) **VERFAHREN ZUR ERZEUGUNG UND/ODER ANORDNUNG VON ZELLKULTUREN**
METHOD FOR PRODUCING AND / OR ARRANGEMENT OF CELL CULTURES
PROCÉDÉ DE GÉNÉRATION ET / OU D'AGENCEMENT DE CULTURES CELLULAIRES

(43) Veröffentlichungstag der Anmeldung: 20.01.2021
(73) Patentinhaber: Axenoll Life Sciences AG, 8001 Zürich (CH)
(72) Erfinder: Deck, David L., 9126 Zumikon (CH); Vasic, Srdan, 9126 Zumikon (CH)
(74) Vertreter: Wenzel Nemetzade Warthmüller Patentanwälte Part mbB

(56) Entgegenhaltungen:
- EP-A1- 2 657 331
- EP-A1- 3 499 393
- EP-A1- 3 505 614
- EP-A2- 3 427 697
- WO-A1-2013/113883
- WO-A1-2014/194180
- US-A1- 2017 348 912

## Beschreibung

Die Erfindung betrifft ein Verfahren zur Erzeugung und/oder Anordnung von Zellkulturen und/oder Zellagglomerationen.

Für die Entwicklung von Zellkulturen ist es bekannt, lebende Zellen in sogenannte Zellträger zu füllen oder aufzutragen. Solche Zellträger können beispielsweise als "Arrays" oder "Hanging Drop Plates" ausgebildet sein. Zellkulturen in Zellträgern können beispielsweise für medizinische oder pharmakologische Untersuchungen herangezogen werden.

Das Befüllen oder Auftragen solcher Zellträger kann beispielsweise mittels Pipetten oder Pipetten-Systemen erfolgen. Hierbei können die Zellen jedoch hoher Scherbelastungen ausgesetzt sein, wodurch die Überlebensrate der Zellen verringert werden kann. Je nach Ausgestaltung der Pipetten oder Pipetten-Systeme kann das Befüllen der Zellträger mit Zellen oder das Auftragen der jeweiligen Zellen auf die Zellträger viele Einzelschritte erfordern, wodurch die gesamte Prozesszeit des Befüllens oder Auftragens verlängert wird. Der verbleibende Untersuchungszeitraum der jeweiligen Zellkultur kann dadurch verringert werden, da bereits mehrere Zellteilungen bis zum Abschluss des Befüllens oder Auftragens erfolgt sein können.

WO 2013/113883 offenbart ein Verfahren zum Herstellen einer dreidimensionalen Struktur aus einem Strukturmaterial enthaltend lebende Zellen, wie z.B. Endothelzellen, Nervenzellen, Osteoblasten, Chondrozyten in einer Flüssigkeit durch Drucken oder Extrudieren.

Vor dem oben dargelegten Hintergrund bestand die Aufgabe der vorliegenden Erfindung darin, ein Verfahren zur Erzeugung und/oder Anordnung von Zellkulturen und/oder Zellagglomerationen anzugeben, das mit verbesserter Produktivität und gleichzeitig verringerter Gefahr von Zellbeschädigungen durchgeführt werden kann. Ebenso bestand die Aufgabe darin, eine Anordnung einer Mehrzahl von Zellkulturen und/oder Zellagglomerationen anzugeben.

In Bezug auf das Verfahren ist diese Aufgabe mit dem Gegenstand des Anspruchs 1 gelöst worden.

Vorteilhafte Ausgestaltungen sind Gegenstand der abhängigen Ansprüche und werden nachfolgend erläutert.

Bei einem erfindungsgemäßen Verfahren zur Erzeugung und/oder Anordnung von Zellkulturen und/oder Zellagglomerationen wird ein lebende Zellen enthaltenes Druckmedium bereitgestellt und das Druckmedium durch ein Drucksieb und/oder eine Druckschablone hindurch gedruckt.

Zellkulturen und/oder Zellagglomerationen, die durch ein erfindungsgemäßes Verfahren erzeugt und/oder angeordnet werden, eignen sich insbesondere für die Durchführung medizinischer und/oder pharmakologischer Untersuchungen. Durch das Drucken des Druckmediums durch ein Drucksieb und/oder eine Druckschablone kann in vorteilhafter Weise ein hohes Maß an Produktivität gewährleistet werden. Insbesondere erlaubt die Ausgestaltung des Drucksiebs und/oder der Druckschablone das Drucken mehrerer definierter Druckbereiche beziehungsweise Druckabschnitte in nur einem Druckschritt. Der gesamte Druckprozess kann somit in einem nur geringen Zeitraum abgeschlossen werden. Der verbleibende Untersuchungszeitraum der jeweiligen Zellkultur und/oder Zellagglomeration kann dadurch verlängert werden.

Gleichzeitig kann das Drucksieb beziehungsweise die Druckschablone in vorteilhafter Weise die Scherbelastungen der Zellen im Druckmedium reduzieren. Insbesondere können Öffnungen des Drucksiebs und/oder der Druckschablone im Hinblick auf verringerte Scherbelastungen in ausreichender Größe ausgestaltet sein.

Die Gefahr von Beschädigungen der Zellen im Druckmedium durch den Vorgang des Druckens beziehungsweise Auftragens kann hierdurch verringert werden. Die Eignung der Zellkultur und/oder Zellagglomeration für medizinische und/oder pharmakologische Untersuchungen wird dadurch verbessert.

Im Sinne der vorliegenden Erfindung kann ein Drucksieb mit einem Gewebe ausgestattet oder durch ein Gewebe und/oder durch einen Rahmen für das Gewebe gebildet sein. Ein solches Gewebe kann für das Hindurchdrücken einer Druckpaste ausgebildet sein. Das Gewebe eines Drucksiebs kann in einem Rahmen angeordnet oder eingespannt sein. Ferner kann ein Gewebe durch Metall- und/oder Kunststofffäden gebildet sein. Solche Metall- und/oder Kunststofffäden können verdrillt angeordnet sein oder einzeln verlaufen.

Zur Erzielung der jeweils gewünschten Druckform kann ein Drucksieb durchlässige und undurchlässige Bereiche aufweisen. Durchlässige Bereiche können für ein Druckmedium durchlässig und undurchlässige Bereiche können für ein Druckmedium undurchlässig sein. Durchlässige Bereiche eines Drucksiebs können im Sinne der vorliegenden Erfindung einen Durchlass und/oder einen Ausschnitt bilden, in dem ein für ein Druckmedium durchlässiges Gewebe angeordnet ist. Undurchlässige Bereiche können ebenfalls mit einem Gewebe versehen sein, welches jedoch für ein Druckmedium undurchlässig ausgestaltet oder zur Erzielung einer Undurchlässigkeit bedeckt ist.

Mit einem Drucksieb können feine Strukturen erzeugt werden, insbesondere mit einer Dicke zwischen 15 µm und 500 µm. Der Druck mittels eines Drucksiebs kann mit verhältnismäßig hoher Genauigkeit erfolgen, insbesondere mit einer hohen Genauigkeit in den Randbereichen des Druckerzeugnisses.

Im Sinne der vorliegenden Erfindung kann eine Druckschablone mit Öffnungen und/oder Ausschnitten ausgestattet sein, insbesondere mit Durchgangsöffnungen und/oder Ausschnitten, die in eine Platte eingebracht sind. Eine Platte mit Öffnungen und/oder Ausschnitten kann eine Druckschablone im Sinne der vorliegenden Erfindung bilden. Öffnungen und/oder Ausschnitte können dementsprechend einen Durchlass für Druckpaste bilden. Durch solche Öffnungen und/oder Ausschnitte kann im Rahmen eines Druckprozesses Druckpaste hindurchgedrückt werden. Eine Druckschablone kann insbesondere frei von Gewebe ausgebildet sein. Insbesondere können die Öffnungen und/oder Ausschnitten einer Druckschablone frei von Gewebe sein.

Gleichzeitig besteht die Möglichkeit, dass innerhalb einer Öffnung und/oder eines Ausschnitts einer Druckschablone ein Formelement angeordnet ist, um die Form der Öffnung und/oder des Ausschnitts entsprechend der jeweils gewünschten Druckform zu definieren. Derartige Formelemente können mit einzelnen Haltefäden oder dergleichen an der gewünschten Position innerhalb der jeweiligen Öffnung und/oder des Ausschnitts gehalten werden.

Druckschablonen eignen sich insbesondere für den Druck von Lagen mit einer Dicke ab 100 µm, insbesondere ab 300 µm, besonders bevorzugt ab 500 µm. Der Druck mittels Druckschablone kann in verhältnismäßig hoher Geschwindigkeit erfolgen, da größere Auftragsstärken zu bewerkstelligen sind.

Gemäß einer vorteilhaften Ausgestaltung kann eine Trägerstruktur für eine Mehrzahl voneinander getrennter Zellkulturen und/oder Zellagglomerationen bereitgestellt werden. Weiterhin kann das Druckmedium durch ein Drucksieb und/oder durch eine Druckschablone auf eine Trägerstruktur gedruckt werden. Die Trägerstruktur kann insbesondere eine Druckunterlage bilden, auf die das Druckmedium nach Durchtritt durch das Drucksieb und/oder durch die Druckschablone gedruckt wird. Eine solche Trägerstruktur kann die Handhabung der Zellkulturen und/oder Zellagglomerationen, insbesondere nach abgeschlossenem Druck vereinfachen.

Es besteht ferner die Möglichkeit, dass eine Trägerstruktur zusammen und/oder abwechselnd mit dem lebende Zellen enthaltenden Druckmedium gedruckt und dadurch bereitgestellt wird. Hierdurch kann ein hohes Maß an Fertigungsflexibilität sichergestellt werden. Die Positionierung und Ausrichtung einer fertigen Trägerstruktur unterhalb eines Drucksiebs und/oder unterhalb einer Druckschablone kann somit entfallen. Der Vorbereitungssaufwand für das Drucken kann somit verringert werden.

Ebenso ist es möglich, dass eine Trägerstruktur vor dem Drucken des lebende Zellen enthaltenes Druckmediums gedruckt und auf diese Weise bereitgestellt wird. Die vollständig gedruckte Trägerstruktur kann dann als Druckunterlage eingesetzt werden.

Gemäß einer weiter vorteilhaften Ausgestaltung können durch den Druck des Druckmediums ein- oder mehrschichtige Zellagglomerationen und/oder ein- oder mehrschichtige Zellkulturen erzeugt werden. Der Druck einer einschichtigen Zellagglomeration und/oder Zellkultur kann mit nur geringem Aufwand und damit zu geringen Kosten realisiert werden. Mehrschichtige Zellagglomerationen und/oder Zellkulturen ermöglichen besonders aussagekräftige Untersuchungen, da die Bedingungen lebender Organismen durch mehrschichtige Ausbildung besonders gut nachgebildet werden können.

In weiter bevorzugter Weise kann durch mehrmaliges Drucken eines lebende Zellen enthaltenden Druckmediums und/oder durch Variation der Zellen beziehungsweise des Zellen aufweisenden Druckmediums in einer Lagenaufbaurichtung (z-Richtung) einfach und schnell ein mehrlagige Gewebe erzeugt werden. Bei einem solchen mehrlagigen Gewebe kann es sich beispielsweise um Haut oder auch um andere Zellgewebearten handeln.

In weiter bevorzugter Weise kann das Druckmedium durch mindestens einen Rakelvorgang und/oder mindestens eine Druckrakelbewegung, insbesondere durch eine Mehrzahl von Rakelvorgängen und/oder eine Mehrzahl von Druckrakelbewegungen, durch das Drucksieb und/oder durch die Druckschablone hindurch und/oder auf eine Trägerstruktur befördert werden. Rakelvorgänge beziehungsweise Druckrakelbewegungen gestatten ein zügiges und gleichmäßigen Drucken des Druckmediums durch das Drucksieb beziehungsweise durch die Druckschablone hindurch. Insbesondere können auf diese Weise besonders reproduzierbare Ergebnisse erzielt werden.

In besonders bevorzugter Weise kann das Druckmedium durch mindestens einen Siebdruckvorgang, insbesondere durch eine Mehrzahl von Siebdruckvorgängen, gedruckt werden. Das Drucken des Druckmediums kann insbesondere im Siebdruckverfahren und/oder im Schablonendruckverfahren erfolgen, besonders bevorzugt im 2D Siebdruckverfahren oder 3D-Siebdruckverfahren. Hierdurch kann ein besonders hohes Maß an Produktivität und Genauigkeit gewährleistet werden. Im Siebdruckverfahren können verhältnismäßig große Volumina eines Druckmediums mit nur geringem Zeit- und Kostenaufwand gedruckt werden. Die Anordnung und/oder Erzeugung von Zellkulturen und Zellagglomerationen kann somit schnell und kostengünstig erfolgen. Gleichzeitig gewährleistet der Einsatz eines Siebdruckverfahrens, insbesondere eines 2D Siebdruckverfahrens oder 3D-Siebdruckverfahrens, eine schonende Verarbeitung des lebende Zellen enthaltenden Druckmediums. Die jeweiligen Zellen können im Siebdruckverfahren mit einer nur geringen Gefahr der Schädigung durch Scherbelastungen verarbeitet und/oder gedruckt werden. Für Drucken des Druckmediums kann insbesondere eine 3D-Siebdruckanlage eingesetzt werden.

In weiter bevorzugter Weise werden Substrukturen der Zellkulturen und/oder Zellagglomerationen beziehungsweise daraus erzeugtes Gewebe bis auf 100 Zellen genau gedruckt, bevorzugt bis auf 50 Zellen, insbesondere 20 Zellen, weiter bevorzugt 10 Zellen, insbesondere bis auf eine einzelne Zelle genau gedruckt. Derartige Druck- beziehungsweise Herstellgenauigkeiten können sowohl in Lagenaufbaurichtung (z-Richtung) und/oder in Richtungen quer zur Lageaufbaurichtung (x/y-Richtungen) erzielt werden. Insbesondere können derartige Genauigkeiten im Wege des 3D-Siebdruckens realisiert werden.

Der Siebdruckvorgang beziehungsweise die Mehrzahl von Siebdruckvorgängen kann in vorteilhafter Weise auf, insbesondere unmittelbar auf, eine Trägerstruktur erfolgen. Das Siebdrucken auf eine Trägerstruktur, beispielsweise auf einen Zellträger, ermöglicht ein hohes Maß an Reproduzierbarkeit sowie Handhabungsfreundlichkeit bei der Entnahme der gedruckten Zellen nach Abschluss des Druckvorgangs.

Es kann ferner von Vorteil sein, wenn das Drucken des lebende Zellen enthaltenden Druckmediums zusammen und/oder abwechselnd mit dem Drucken der Trägerstruktur im 2D Siebdruckverfahren oder 3D-Siebdruckverfahren erfolgt. Dies ermöglicht eine flexible Ausgestaltung der Trägerstruktur sowie des gesamten Druckverfahrens. Die jeweils gewünschte Form der Trägerstruktur kann somit bedarfsorientiert erzeugt werden.

In noch weiter bevorzugter Ausgestaltung kann durch das Bedrucken einer Trägerstruktur mit dem Druckmedium eine dreidimensionale Zellkultur und/oder Zellagglomeration erzeugt werden. Dreidimensionale Zellkulturen und/oder Zellagglomerationen lassen sich insbesondere durch mehrmaliges Drucken beziehungsweise durch 3D-Siebdruck erzeugen.

Ferner ist es möglich, dass sich nach dem Bedrucken einer Trägerstruktur mit dem Druckmedium die jeweilige Zellkultur und/oder Zellagglomeration zu einer Zellkultur und/oder Zellagglomeration, insbesondere einer dreidimensionalen Zellkultur und/oder Zellagglomeration, entwickelt. Dementsprechend kann zwischen dem Abschluss des Druckens und der Entwicklung einer Zellkultur beziehungsweise Zellagglomeration eine Entwicklungszeit erforderlich sein. Über die Dauer einer solchen Entwicklungszeit können die Eigenschaften und/oder Beschaffenheiten der Zellkulturen beziehungsweise Zellagglomerationen beeinflusst werden.

Gemäß einer weiter bevorzugten Ausgestaltung können in den Druckabschnitten, also den durch Druck des lebenden Zellen enthaltenden Druckmediums erzeugten Abschnitten, strukturelle Gerüste und/oder Scaffolds vorgesehen sein. Ebenso können strukturelle Gerüste und/oder Scaffolds gemeinsam mit dem Druckmedium gedruckt werden und/oder in diesem enthalten sein. Gerüste und/oder Scaffolds eignen sich in besonders vorteilhafter Weise für die Erzeugung dreidimensionaler Strukturen. Zellkulturen und/oder Zellagglomerationen können auf diese Weise mit nur geringem Aufwand und gleichzeitig reproduzierbar dreidimensional ausgebildet werden.

Beim Drucken des lebende Zellen aufweisenden Druckmediums können in besonders vorteilhafter Weise Kavitäten, Aussparungen, linienförmige oder kanalförmige Strukturen vorgesehen werden. Derartige Formen erlauben eine verbesserte Funktionalisierung der mit dem Druckmedium erzeugten Zellkulturen, Zellagglomerationen und/oder Geweben. Es können durch solche Kavitäten, Aussparungen und/oder Strukturen beispielsweise Scaffolds und/oder Blutgefäße ausgebildet werden.

In besonders bevorzugter Weise kann eine Kombination von Materialien gedruckt werde, insbesondere um Scaffolds und/oder Matrizes mit Zellen in einem einzigen Druckprozess mit nur geringem Aufwand herzustellen.

Nach einer weiter bevorzugten Ausgestaltung kann sich die jeweilige Zellkultur und/oder die Zellagglomeration nach dem Drucken zu Gewebe entwickeln. Die Entwicklung von Gewebe kann für die Nutzung in medizinischen oder pharmakologischen Untersuchungen besonders von Vorteil sein. Ebenso kann derart entwickeltes Gewebe zur medizinischen Behandlung eingesetzt werden. Schließlich kann derart entwickeltes Gewebe als Lebensmittel dienen. Die jeweilige Zellkultur und/oder die Zellagglomeration kann sich nach dem Drucken insbesondere zu synthetischem Fleisch entwickeln, das zum Verzehr geeignet sein kann.

Gemäß einer weiteren vorteilhaften Ausgestaltung kann die Trägerstruktur für die Anordnung und/oder Aufnahme einer Mehrzahl voneinander getrennter Zellkulturen und/oder Zellagglomeration ausgebildet sein. Die Trägerstruktur kann insbesondere vordefinierte Anordnungs- und/oder Aufnahmeabschnitte für voneinander getrennte Zellkulturen und/oder Zellagglomeration aufweisen. Hierdurch kann eine sichere und definierte Anordnung der gedruckten Zellen beziehungsweise durch die Zellen gebildete Zellkulturen und/oder Zellagglomeration auf der Trägerstruktur gewährleistet werden.

In bevorzugter Weise können die Anordnungs- und/oder Aufnahmeabschnitte in mehreren Zeilen und Reihen auf der Trägerstruktur vorgesehen sein. Insbesondere kann sich die Anzahl der Zeilen von der Anzahl an Reihen unterscheiden. Die äußere Form der Trägerstruktur kann an die Anzahl der Zeilen und Reihen angepasst sein. Weiter bevorzugt kann die Trägerstruktur Vertiefungen zur Aufnahme des Druckmediums aufweisen und/oder die Anordnungs- und/oder Aufnahmeabschnitte können als Vertiefung ausgebildet sein.

In besonders bevorzugter Weise kann die Trägerstruktur als Objektträger und/oder Zellkulturplatte und/oder Zellträger ausgebildet sein. Die Trägerstruktur eignet sich damit in besonders vorteilhafter Weise für die Verwendung und/oder Handhabung in Laboratorien beziehungsweise für standardisierte Transportbehältnisse, Transportvorrichtungen und/oder Aufbewahrungsvorrichtungen.

In weiter bevorzugter Weise kann das Drucksieb und/oder die Druckschablone eine Mehrzahl von Ausschnitten für den Durchlass des Druckmediums aufweisen, insbesondere getrennt voneinander ausgebildete Ausschnitte. Es lässt sich damit ein definierter Durchtritt des Druckmediums durch das Drucksieb und/oder die Druckschablone realisieren. Insbesondere kann das Druckmedium hierdurch in definierter Weise an unterschiedlichen und/oder voneinander beabstandeten Positionen gedruckt und/oder auf eine Trägerstruktur aufgebracht werden.

In weiter bevorzugter Weise können die Ausschnitte des Drucksiebs und/oder der Druckschablone mit vordefinierten Anordnungs- und/oder Aufnahmeabschnitten der Trägerstruktur korrespondieren. Hierdurch kann das Druckmedium gezielt in und/oder auf die Anordnungs- und/oder Aufnahmeabschnitte der Trägerstruktur gedruckt werden. Insbesondere kann durch eine solche Ausgestaltung vermieden werden, dass ein versehentliches oder unerwünschtes Bedrucken der Trägerstruktur außerhalb der Anordnungs- und/oder Aufnahmeabschnitte erfolgt. Gleichzeitig kann hierdurch ein hohes Druckvolumen pro Druckvorgang realisiert werden.

Gemäß einer weiter bevorzugten Ausgestaltung kann zumindest ein Ausschnitt und/oder Durchlass des Drucksiebs und/oder der Druckschablone für den Durchlass des Druckmediums eine Größe und/oder einen Durchmesser von wenigstens 1 mm aufweisen, insbesondere von wenigstens 2, bevorzugt von wenigstens 2 mm, bevorzugt von wenigstens 3 mm, weiter bevorzugt von 4 mm, weiter bevorzugt von wenigstens 5 mm.

Gemäß einer noch weiter bevorzugten Ausgestaltung kann zumindest ein Ausschnitt und/oder Durchlass des Drucksiebs und/oder der Druckschablone für den Durchlass des Druckmediums eine Größe und/oder einen Durchmesser von bis zu 100 mm aufweisen, insbesondere von bis zu 50 mm, bevorzugt von bis zu 40 mm, bevorzugt von bis zu 30 mm, weiter bevorzugt von bis zu 25 mm, weiter bevorzugt von bis zu 20 mm, noch weiter bevorzugt von bis zu 15 mm, weiter bevorzugt von bis zu 10 mm, noch weiter bevorzugt von bis zu 9 mm, weiter bevorzugt von bis zu 8 mm, noch weiter bevorzugt von bis zu 5 mm.

Gemäß einer weiter bevorzugten Ausgestaltung kann zumindest ein Ausschnitt und/oder Durchlass des Drucksiebs und/oder der Druckschablone für den Durchlass des Druckmediums eine Größe und/oder einen Durchmesser von 1 mm bis 100 mm aufweisen, insbesondere von 2 mm bis 50 mm, bevorzugt von 2 mm bis 40 mm, bevorzugt von 2 mm bis 30 mm, weiter bevorzugt von 3 mm bis 30 mm, weiter bevorzugt von 3 mm bis 25 mm, weiter bevorzugt von 3 mm bis 20 mm, noch weiter bevorzugt von 4 mm bis 20 mm, weiter bevorzugt von 4 mm bis 15 mm, weiter bevorzugt von 4 mm bis 10 mm, noch weiter bevorzugt von 4 mm bis 9 mm, weiter bevorzugt von 4 mm bis 8 mm, noch weiter bevorzugt von 5 mm bis 10 mm, weiter bevorzugt von 5 mm bis 8 mm. Durch einen derart bemessenen Ausschnitt und/oder Durchlass im Drucksieb und/oder in der Druckschablone kann einerseits eine nur geringe Scherbelastung der lebenden Zellen im Druckmedium und gleichzeitig auch ein verhältnismäßig großes Druckvolumen je Druckvorgang sichergestellt werden.

Gemäß einer bevorzugten Ausgestaltung kann eine Mehrzahl von Anordnungs- und/oder Aufnahmeabschnitten der Trägerstruktur zeitgleich und/oder in einem Druckvorgang und/oder durch einen Rakelvorgang mit dem Druckmedium bedruckt und/oder befüllt werden. Dies kann mit nur geringem Aufwand bewerkstelligt werden. Insbesondere können in einem Druckvorgang sämtliche Anordnungs- und/oder Aufnahmeabschnitte der Trägerstruktur mit dem Druckmedium bedruckt werden. Das sequentielle Befüllen einzelner Aufnahmeabschnitte in vielen Einzelschritten kann somit vermieden werden.

Es ist weiterhin möglich, dass unterschiedliche Anordnungs- und/oder Aufnahmeabschnitte der Trägerstruktur in zeitlicher Abfolge entsprechend ihres Abstands zueinander sowie entsprechend der jeweiligen Rakelgeschwindigkeit bedruckt und/oder befüllt werden. Die Einzelnen Anordnungs- und/oder Aufnahmeabschnitte der Trägerstruktur können somit entsprechend einer Rakelbewegung befüllt werden. Eine unmittelbar zeitgleiche Befüllung der Anordnungs- und/oder Aufnahmeabschnitte kann somit unterbleiben, wodurch die Verfahrensflexibilität erhöht wird.

Gemäß einer weiter bevorzugten Ausführungsform des erfindungsgemäßen Verfahrens kann das Druckmedium an vereinzelten Positionen auf eine Trägerstruktur gedruckt werden. Durch das Bedrucken einer Trägerstruktur mit dem Druckmedium können in besonders vorteilhafter Weise voneinander unabhängige und/oder fluidtechnisch getrennte Druckabschnitte erzeugt werden. In der Folge lassen sich mehrere voneinander unabhängige Zellkulturen und/oder Zellagglomerationen erzeugen, die für eine verhältnismäßig große Anzahl an medizinischen und/oder pharmakologischen Untersuchungen zur Verfügung stehen.

Es kann weiter von Vorteil sein, während des Druckens, vor dem Drucken und/oder nach dem Drucken eine Überlagerung des Druckmediums mit einem sterilem Gasmedium vorzunehmen, insbesondere mit steriler Luft. Sterilitätsanforderungen an den Umgang mit und/oder die Verarbeitung von lebenden Zellen können auf diese Weise sicher befolgt werden. Eine unerwünschte Verunreinigung der Zellkulturen und/oder Zellagglomerationen kann somit vermieden werden.

Bei dem Druckmedium kann es sich insbesondere um ein Nährmedium und/oder ein Nährstoffe enthaltendes Medium, insbesondere eine Nährflüssigkeit, handeln. Eine solches Druckmedium beziehungsweise Nährmedium kann insbesondere als sogenannte Bio-Ink ausgebildet sein.

Gemäß einer noch weiteren bevorzugten Ausgestaltung kann das Druckmedium als Druckpaste und/oder niederviskose oder mittelviskose Nährflüssigkeit und/oder flüssige Suspension ausgebildet sein. In rein gel-förmigen Medien können Zellen und/oder Nährstoffe nur in eingeschränkter Weise diffundieren, was dazu führen kann, dass die Verfügbarkeit der Nährstoffe eingeschränkt ist und somit auch die Lebensdauer der Zellen. Durch die Ausbildung des Druckmedium als verhältnismäßig dünnflüssige Druckpaste und/oder niederviskose oder mittelviskose Nährflüssigkeit und/oder flüssige Suspension kann ein hohes Maß an Nährstoffversorgung der lebenden Zellen innerhalb des Druckmediums sichergestellt werden.

Ebenso ist es möglich, dass das Druckmedium gelförmig und/oder als hochviskoses Nährmedium ausgebildet ist, wodurch die Verarbeitung beziehungsweise das Drucken vereinfacht werden kann.

Ferner kann das Druckmedium als Sol-Gel Matrix ausgebildet sein und/oder eine variable Viskosität aufweisen und/oder die Viskosität des Druckmediums kann durch einen Trocknungsschritt und/oder Temperierungsschritt, insbesondere einer Temperaturverringerung oder Temperaturerhöhung, veränderbar sein. Je nach erfolgtem Verarbeitungsschritt kann die Viskosität damit verändert werden. Es kann hierdurch über verhältnismäßig lange Zeiträume ein hohes Maß an Nährstoffversorgung gewährleistet werden und gleichzeitig kann die Viskosität zwecks Verarbeitung oder Weiterverarbeitungsschritten verändert werden.

Das Druckmedium kann weiterhin strukturviskos und/oder scherverdünnend ausgebildet sein. Bei verhältnismäßig hohen Scherbelastungen des Druckmediums kann sich hierdurch eine temporär geringere Viskosität einstellen und dadurch die Scherbelastung verringert werden. Die Gefahr von Zellbeschädigungen wird dadurch weiter verringert.

In weiter bevorzugter Weise kann zwischen aufeinanderfolgenden Druckschritten ein Trocknungs- und/oder Temperierungsschritt des aufgedruckten Druckmediums erfolgen. Es ist ebenso möglich, dass nach dem Drucken des Druckmediums ein Sol-Gel-Übergang erzeugt wird, insbesondere durch einen Trocknungs- und/oder Temperierungsschritt, und/oder dass nach einem erzeugten Sol-Gel-Übergang ein weiterer Druckvorgang vorgenommen wird. Die Eigenschaften des Druckmediums beziehungsweise der dadurch erzeugten Zellkultur und/oder Zellagglomeration lassen sich hierdurch gezielt in Abhängigkeit des jeweils erfolgten Verfahrensstadiums beeinflussen.

In weiter bevorzugter Weise kann das Druckmedium beim Drucken flüssig sein oder eine geringe Viskosität aufweisen, sodass ein einfaches Drucken möglich ist. Im Anschluss an den Druck beziehungsweise den Druck einer Drucklage kann die die jeweilige Drucklage verfestigt beziehungsweise die Viskosität des gedruckten Mediums erhöht werden, beispielsweise durch Temperierung und/oder durch Erzeugung eines Sol-Gel Übergangs. Das jeweilige Druckobjekt beziehungsweise die durch Drucken erzeugte Zellkultur und/oder Zellagglomeration kann hierdurch in geeigneter Weise in einer Lagenaufbaurichtung (z-Richtung) aufgebaut werden.

Sobald das jeweilige Druckobjekt in der Höhe (z-Richtung) fertig aufgebaut ist, kann dieses in eine geeignete Form und/oder Schablone eingesetzt werden oder die Form und/oder Schablone kann auf das Druckobjekt aufgesetzt werden, um dieses zu umgeben. Das Druckobjekt kann somit in x/y- Richtung begrenzt werden. Das gedruckte Medium beziehungsweise die durch Drucken erzeugte Zellkultur und/oder Zellagglomeration kann wieder in einen flüssigeren Zustand beziehungsweise in einen Zustand mit geringerer Viskosität gebracht werden, ohne dass es zu einem zerlaufen kommt. Hierdurch können Nährstoffe innerhalb der durch Drucken erzeugten Zellkultur und/oder Zellagglomeration wieder frei zirkulieren. Gewebe kann sich in verbesserter Weise entwickeln.

In weiter bevorzugter Ausgestaltung kann das Druckmedium teilungsfähige und/oder zur Teilungsfähigkeit induzierbare Zellen enthalten. Ferner kann es sich bei den lebenden Zellen des Druckmediums um menschliche, tierische und/oder pflanzliche Zellen handeln. Insbesondere kann das Druckmedium alle Arten von menschlichen, tierischen und/oder pflanzlichen Zellen enthalten. Besonders bevorzugt kann es sich bei den lebenden Zellen um alle teilungsfähigen oder zur Teilungsfähigkeit induzierbaren Zellen des menschlichen oder tierischen Körpers oder von Pflanzen handeln. Es ergibt sich hierdurch eine besondere Eignung für medizinische und pharmakologische Untersuchungen.

In weiter bevorzugter Weise kann das Druckmedium lebende Zellen aus der Gruppe der Primärzellen aufweisen, insbesondere aller Arten von menschlichen, tierischen und/oder pflanzlichen Primärzellen.

Ebenso kann das Druckmedium lebende Zellen aus der Gruppe der Zelllinien aufweisen, insbesondere aller Arten von menschlichen, tierischen und/oder pflanzlichen etablierten Zelllinien.

Bei Primärzellen kann es sich beispielsweise handeln um Organzellen, insbesondere Organzellen aller Organe, Hautzellen, Fibroblasten, Chondroblasten, Osteoblasten, Muskelzellen, Herzmuskelzellen, Nervenzellen, Leberzellen, Inselzellen, vaskuläre Zellen, Drüsengewebezellen, Tumorzellen, insbesondere Tumorzellen aller Tumorgewebe, Stammzellen, insbesondere hämatopoetische, mesenchymale und/oder neuronale Stammzellen und/oder induzierte pluripotente Stammzellen aus Geweben, wie beispielsweise Fettgewebe, Haut und/oder Nabelschnur, und/oder totipotente Stammzellen, insbesondere Eizellen und/oder Embryonalzellen, und/oder pluripotente, multipotente, oligopotente und/oder unipotente Stammzellen, Blutzellen und/oder Immunzellen.

Bei Zelllinien kann es sich beispielsweise um immortalisierte Zellen der voranstehend benannten Zelltypen und/oder Zellen der voranstehend genannten Gewebeypen handeln.

Es kann weiter von Vorteil sein, wenn das Druckmedium unterschiedliche Zellen und/oder Zellentypen enthält, insbesondere unterschiedliche der voranstehend benannten Zellen und/oder Zellentypen. Es kann sich hierdurch eine weiter verbesserte Eignung der erzeugten Zellkulturen und/oder Zellagglomerationen für medizinische und/oder pharmakologische Untersuchungen ergeben.

Weiter bevorzugt kann ein aus dem Druckmedium erzeugtes Gewebe unterschiedliche Zellen und/oder Zellentypen enthalten, insbesondere unterschiedliche der voranstehend benannten Zellen und/oder Zellentypen. Es kann sich hierdurch ebenfalls eine weiter verbesserte Eignung des erzeugten Gewebes für medizinische und/oder pharmakologische Untersuchungen ergeben.

Es kann weiter von Vorteil sein, wenn das Druckmedium zumindest einen Wachstumsfaktor und/oder zumindest ein Protein und/oder extrazelluläres Matrixprotein enthält, insbesondere ein Wachstumsfaktor und/oder Protein aus der Gruppe der Zytokine, insbesondere als Wachstumsregulatoren, Interferone und/oder Interleukine, Membranbestandteile, Laminine, Kollagene, insbesondere Kollagen Typ 4, Proteoglykane, Entactine, Nidogene, Zelladhärenzfaktoren, insbesondere Fibronectin und/oder Vitronectin, Wachstumsfaktoren, insbesondere der EGF-Familie, der TGF-Familie, PDGF, VEGF, Somatomedine, insbesondere IGF, NGF, PTGF, und/oder Schutzfaktoren, insbesondere gewebespezifische Plasminogenaktivatoren, Serumalbumine und/oder CMC. In besonderes bevorzugter Weise kann das Druckmedium zumindest eine oder mehrere dieser Substanzen aus den vorstehend benannten Gruppen aufweisen. Es kann sich hierdurch eine weiter verbesserte Eignung der mittels des Druckmediums erzeugten Zellkulturen, Zellagglomerationen und/oder Geweben für die Durchführung von medizinischen und/oder pharmakologischen Untersuchungen ergeben.

In weiter bevorzugter Weise kann das Druckmedium Zellen mit einer Größe von 5-50µm, 5-40µm, 10-50µm, 10-40µm, 10-30µm, 20-40µm, 25-40µm, 25-30µm im Durchmesser oder in der Querschnittslänge aufweisen. Ferner kann eine Zellenkultur eine Anzahl von 5 bis 1000 Zellen aufweisen, insbesondere von 10 bis 1000 Zellen, 20 bis 500 Zellen, 50 bis 500 Zellen, 100 bis 500 Zellen, 200 bis 500 Zellen oder 300 bis 500 Zellen. Hierdurch können besonders aussagekräftige Untersuchungen mittels der durch das Druckmedium erzeugten Zellkulturen und/oder Zellagglomerationen vorgenommen werden.

Ein weiterer unabhängiger Aspekt der vorliegenden Erfindung betrifft ein Verfahren zur von Erzeugung und/oder Anordnung von Zellkulturen und/oder Zellagglomerationen bei dem eine Trägerstruktur für eine Mehrzahl voneinander getrennter Zellkulturen bereitgestellt und ein lebende Zellen enthaltenes Druckmedium mittels Siebdrucks auf die Trägerstruktur gedruckt wird.

Ein noch weiterer unabhängiger Aspekt der vorliegenden Erfindung betrifft eine Anordnung einer Mehrzahl von Zellkulturen und/oder Zellagglomerationen, die durch ein voranstehend beschriebenes Verfahren auf eine Trägerstruktur gedruckt sind.

Ein noch weiterer unabhängiger Aspekt der vorliegenden Erfindung betrifft eine Anordnung einer Mehrzahl von Geweben und/oder Gewebeabschnitten, die zumindest durch Drucken eines Druckmediums gemäß einem voranstehend beschriebenen Verfahren auf eine Trägerstruktur und anschließende und/oder vorangehende Zellkultivierung erzeugt sind.

Gemäß einer besonders bevorzugten Ausgestaltung können die Zellenkulturen und/oder Zellagglomerationen und/oder Geweben hinsichtlich des metabolischen Zustands der Zellen, des Zellalter und/oder der Zellenanzahl und/oder der Dimension der Zellenanzahl gleich sein. Ebenso ist es möglich, dass bei den Zellenkulturen und/oder Zellagglomerationen und/oder Geweben Abweichungen hinsichtlich des metabolischen Zustands der Zellen, des Zellalters und/oder Zellenanzahl geringer sind als 20%, insbesondere geringer als 10%, geringer als 5%, geringer als 3%, geringer als 2% oder geringer als 1%. Durch eine derartige Ausgestaltung der Anordnung können die Zellenkulturen und/oder Zellagglomerationen und/oder Geweben für eine Vielzahl von Untersuchungen dienen, in denen die Untersuchungsparameter zwischen den einzelnen Zellenkulturen und/oder Zellagglomerationen und/oder Geweben in geeigneter Weise variiert werden können. Solche Untersuchungsreihen können besonders Aussagekräftig sein.

Vorliegend kann unter Gewebe insbesondere Zellgewebe verstanden werden.

Weitere Ausgestaltungen der vorliegenden Erfindung ergeben sich aus Kombinationen der in den Ansprüchen, der Beschreibung sowie den Figuren offenbarten Merkmale. Die vorliegende Erfindung wird nachfolgend anhand von Ausführungsbeispielen und zugehörigen Zeichnungen erläutert.

Es zeigen jeweils schematisch:
- Fig. 1: eine Trägerstruktur für Zellkulturen und/oder Zellagglomerationen und ein darüber angeordnetes Drucksieb beziehungsweise eine Druckschablone in Perspektivdarstellung,
- Fig. 2: eine Anordnung einer Mehrzahl von Zellkulturen und/oder Zellagglomerationen auf einer Trägerstruktur gemäß Fig. 1 in Perspektivdarstellung.

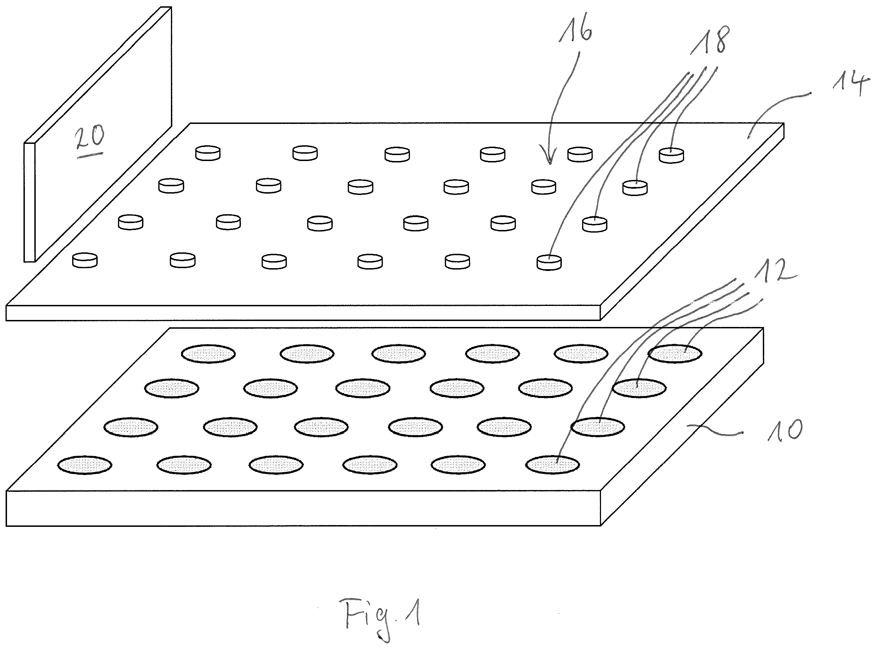

In Fig. 1 ist eine Trägerstruktur 10 für Zellkulturen und/oder Zellagglomerationen in Perspektivdarstellung gezeigt. Bei der Trägerstruktur 10 kann es sich insbesondere um eine Zellkulturplatte, um einen Objektträger beziehungsweise um ein sogenanntes Array für Zellkulturen und/oder Zellagglomerationen handeln. Die Trägerstruktur 10 kann eine Mehrzahl von Aufnahmeabschnitten 12 aufweisen.

Die Aufnahmeabschnitte 12 können zur Aufnahme von hier nicht näher dargestellten Zellkulturen und/oder Zellagglomerationen ausgebildet sein. Insbesondere kann ein hier nicht näher dargestelltes lebende Zellen enthaltendes Druckmedium durch Drucken in die Aufnahmeabschnitte 12 befördert werden, was nachstehend näher erläutert wird. Sobald ein solches Druckmedium in den Aufnahmeabschnitten 12 angeordnet ist, kann sich dieses zu Zellkulturen und/oder Zellagglomerationen entwickeln. Ebenso kann eine bereits aus Zellkulturen und/oder Zellagglomerationen bestehendes und/oder Zellkulturen und/oder Zellagglomerationen enthaltendes Druckmedium durch Drucken in den Aufnahmeabschnitten 12 angeordnet werden.

Gemäß der beispielhaften Ausgestaltung in Fig. 1 können die Aufnahmeabschnitte 12 durch Vertiefungen gebildet sein, insbesondere durch schalenförmige und/oder halbkugelförmige und/oder zylindrische und/oder rechteckige Vertiefungen. Die in Fig. 1 beispielhaft gezeigte Trägerstruktur 10 weist insgesamt 24 Aufnahmeabschnitte 12 auf. Die Aufnahmeabschnitte 12 können beispielhaft in insgesamt 4 Reihen und 6 Zeilen in der Trägerstruktur 10 ausgebildet sein. Es können selbstverständlich auch Trägerstrukturen 10 mit einer anderen Anzahl an Aufnahmeabschnitten 12 beziehungsweise mit anderen Anordnungen von Aufnahmeabschnitten 12 geeignet sein.

In der Fig. 1 ist ferner ein Druckschablone 14 in schematischer Perspektivdarstellung gezeigt. Die Druckschablone 14 kann durch eine Platte 16 gebildet sein, die eine Mehrzahl von Ausschnitten 18 für den Durchlass eines Druckmediums aufweist. Die Ausschnitte 18 sind insbesondere getrennt voneinander ausgebildet.

Anstelle der Druckschablone 14 kann auch ein hier nicht näher dargestelltes Drucksieb vorgesehen sein, das mit einem Gewebe ausgestattet ist. Innerhalb eines solchen Gewebes können Bereiche vorgesehen sein, die für Druckpaste durchlässig sind und von undurchlässigen Bereichen begrenzt werden. Durchlässige Bereiche werden vorliegend ebenfalls als Ausschnitt bezeichnet, obwohl diese ein Gewebe aufweisen können.

Die Ausschnitte 18 der Druckschablone 14 oder eines hier nicht gezeigten Drucksiebs können beispielsweise einen Durchmesser von wenigstens 1 mm, insbesondere von wenigstens 2 mm, bevorzugt von wenigstens 3 mm, weiter bevorzugt von wenigstens 4 mm, weiter bevorzugt von wenigstens 5 mm, aufweisen.

Die Ausschnitte 18 der Druckschablone 14 oder eines hier nicht gezeigten Drucksiebs können insbesondere einen Durchmesser von 1 mm bis 100 mm, insbesondere von 2 mm bis 50 mm, bevorzugt von 2 mm bis 40 mm, bevorzugt von 2 mm bis 30 mm, weiter bevorzugt von 3 mm bis 30 mm, weiter bevorzugt von 3 mm bis 25 mm, weiter bevorzugt von 3 mm bis 20 mm, noch weiter bevorzugt von 4 mm bis 20 mm, weiter bevorzugt von 4 mm bis 15 mm, weiter bevorzugt von 4 mm bis 10 mm, noch weiter bevorzugt von 4 mm bis 9 mm, weiter bevorzugt von 4 mm bis 8 mm, noch weiter bevorzugt von 5 mm bis 10 mm, weiter bevorzugt von 5 mm bis 8 mm, aufweisen. Die Ausschnitte 18 der Druckschablone 14 können unterschiedliche Durchmesser oder auch identische Durchmesser aufweisen.

Ferner können die Ausschnitte 18 der Druckschablone 14 oder eines hier nicht gezeigten Drucksiebs in besonders bevorzugter Weise mit den vordefinierten Aufnahmeabschnitten 12 der Trägerstruktur 10 korrespondieren.

Gemäß der vorliegenden Erfindung wird ein lebende Zellen enthaltenes Druckmedium durch ein Drucksieb und/oder durch die Druckschablone 14 gedruckt.

Für das Drucken des Druckmediums kann das Drucksieb und/oder die Druckschablone 14 oberhalb der Trägerstruktur 10 angeordnet werden, wie in Fig. 1 gezeigt. Das ferner in Fig. 1 schematisch gezeigte Druckrakel 20 kann für das Drucken eines Druckmediums durch das Drucksieb und/oder durch die Druckschablone 14 eingesetzt werden. Dabei kann das Druckmedium durch mindestens einen Rakelvorgang und/oder mindestens eine Druckrakelbewegung, insbesondere durch eine Mehrzahl von Rakelvorgängen des Druckrakels 20 und/oder eine Mehrzahl von Druckrakelbewegungen des Druckrakels 20, durch das Drucksieb und/oder die Druckschablone 14 hindurch gedruckt werden. Hierdurch lässt sich das Druckmedium besonders zuverlässig auf die Trägerstruktur 10 befördern.

Das Drucken des Druckmediums mittels des Drucksiebs und/oder der Druckschablone 14 und/oder des Druckrakels 20 kann insbesondere im Siebdruckverfahren erfolgen. Es kann sich bei dem Siebdruckverfahren insbesondere um ein 2D-Siebdruckverfahren oder 3D-Siebdruckverfahren handeln.

Es besteht ferner die Möglichkeit, dass die Trägerstruktur 10 zusammen und/oder abwechselnd mit dem lebende Zellen enthaltenden Druckmedium gedruckt wird. Insbesondere kann das Drucken des lebende Zellen enthaltenden Druckmediums zusammen und/oder abwechselnd mit dem Drucken der Trägerstruktur 10 im 3D-Siebdruckverfahren erfolgen. Ferner kann die Trägerstruktur 10 vor dem Drucken des lebende Zellen enthaltenes Druckmediums gedruckt werden, insbesondere im 3D-Siebdruckverfahren. Ebenso kann die Trägerstruktur 10 anderweitig hergestellt sein und vor dem Drucken des lebende Zellen enthaltenes Druckmediums bereitgestellt werden.

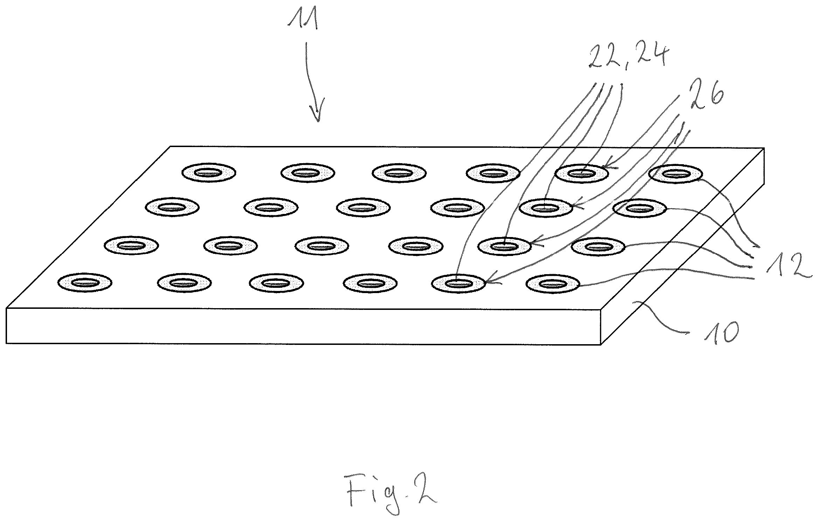

In Fig. 2 ist eine Anordnung 11 einer Mehrzahl von Zellkulturen 22 und/oder Zellagglomerationen 24 auf einer Trägerstruktur 10 in Perspektivdarstellung gezeigt. Jede der Zellkulturen 22 und/oder Zellagglomerationen 24 ist in einem der Aufnahmeabschnitte 12 angeordnet. Durch den Druck des Druckmediums können ein- oder mehrschichtige Zellkulturen 22 und/oder ein- oder mehrschichtige Zellagglomerationen 24 erzeugt werden, was hier nicht im Einzelnen dargestellt ist. Ferner kann durch das Bedrucken der Trägerstruktur 10 mit dem Druckmedium eine dreidimensionale Zellkultur 22 und/oder Zellagglomeration 24 erzeugt werden. Nach dem Bedrucken der Trägerstruktur 10 mit dem Druckmedium kann sich die jeweilige Zellkultur 22 und/oder Zellagglomeration 24 zu einer dreidimensionalen Zellkultur 22 und/oder Zellagglomeration 24 entwickeln.

Es besteht ferner die Möglichkeit, dass in Druckabschnitten 26 beziehungsweise Aufnahmeabschnitten 12 strukturelle Gerüste und/oder Scaffolds vorgesehen sind, die hier ebenfalls nicht näher dargestellt sind. Solche strukturellen Gerüste und/oder Scaffolds können gemeinsam mit dem Druckmedium gedruckt werden und/oder in diesem enthalten sein.

In besonders bevorzugter Weise kann eine Mehrzahl von Aufnahmeabschnitten 12 der Trägerstruktur 10 zeitgleich und/oder in einem Druckvorgang und/oder durch einen Rakelvorgang mit dem Druckmedium bedruckt und/oder befüllt werden. Ebenso können unterschiedliche Aufnahmeabschnitte 12 der Trägerstruktur 10 in zeitlicher Abfolge entsprechend ihres Abstands zueinander sowie entsprechend der jeweiligen Rakelgeschwindigkeit bedruckt und/oder befüllt werden.

Durch das Bedrucken der Trägerstruktur 10 mit dem Druckmedium können voneinander unabhängige und/oder fluidtechnisch getrennte Druckabschnitte 26 erzeugt werden. Insbesondere kann je Aufnahmeabschnitt 12 ein Druckabschnitt 26 erzeugt werden. Ein Druckabschnitt 26 kann eine Zellkultur 22 und/oder eine Zellagglomeration 24 bilden oder sich zu einer Zellkultur 22 und/oder eine Zellagglomeration 24 entwickeln. Ferner können sich die Zellkulturen 22 und/oder die Zellagglomerationen 24 nach dem Drucken zu Gewebe entwickeln.

Bei der in Fig. 2 gezeigten Anordnung 11 können die einzelnen Zellenkulturen 22 und/oder Zellagglomerationen 24 hinsichtlich des metabolischen Zustands der Zellen, des Zellalters und/oder der Zellenanzahl und/oder der Dimension der Zellenanzahl gleich sein. Ebenso besteht die Möglichkeit, dass in der Anordnung 11 gemäß Fig. 2 Abweichungen hinsichtlich des metabolischen Zustands der Zellen, des Zellalters und/oder der Zellenanzahl geringer als 20%, insbesondere geringer als 10%, geringer als 5%, geringer als 3%, geringer als 2% oder geringer als 1% sind.

Das Druckmedium beziehungsweise die damit erzeugten Zellenkulturen 22 und/oder Zellagglomerationen 24 können teilungsfähige und/oder zur Teilungsfähigkeit induzierbare Zellen enthalten. Es kann sich bei den lebenden Zellen des Druckmediums beziehungsweise der Zellenkulturen 22 und/oder Zellagglomerationen 24 um menschliche, tierische und/oder pflanzliche Zellen handeln. Insbesondere kann/können das Druckmedium beziehungsweise die Zellenkulturen 22 und/oder Zellagglomerationen 24 alle Arten von menschlichen, tierischen und/oder pflanzlichen Zellen enthalten. Besonders bevorzugt kann es sich bei den lebenden Zellen des Druckmediums beziehungsweise der Zellenkulturen 22 und/oder Zellagglomerationen 24 um alle teilungsfähigen oder zur Teilungsfähigkeit induzierbaren Zellen des menschlichen oder tierischen Körpers oder von Pflanzen handeln.

Ferner kann das Druckmedium beziehungsweise eine daraus erzeugte Zellkultur 22 und/oder Zellagglomeration 24 lebende Zellen aus der Gruppe der Primärzellen aufweisen, insbesondere alle Arten von menschlichen, tierischen und/oder pflanzlichen Primärzellen. Ebenso kann das Druckmedium beziehungsweise eine daraus erzeugte Zellkultur 22 und/oder Zellagglomeration 24 lebende Zellen aus der Gruppe der Zelllinien aufweisen, insbesondere alle Arten von menschlichen, tierischen und/oder pflanzlichen etablierten Zelllinien. Das Druckmedium beziehungsweise eine daraus erzeugte Zellkultur 22 und/oder Zellagglomeration 24 kann schließlich unterschiedliche Zellen und/oder Zellentypen enthalten.

Ferner kann das Druckmedium beziehungsweise eine daraus erzeugte Zellkultur 22 und/oder Zellagglomeration 24 zumindest einen Wachstumsfaktor und/oder zumindest ein Protein und/oder extrazelluläres Matrixprotein enthalten, insbesondere ein Wachstumsfaktor und/oder Protein aus der Gruppe der Zytokine, insbesondere als Wachstumsregulatoren, Interferone und/oder Interleukine, Membranbestandteile, Laminine, Kollagene, insbesondere Kollagen Typ 4, Proteoglykane, Entactine, Nidogene, Zelladhärenzfaktoren, insbesondere Fibronectin und/oder Vitronectin, Wachstumsfaktoren, insbesondere der EGF-Familie, der TGF-Familie, PDGF, VEGF, Somatomedine, insbesondere IGF, NGF, PTGF, und/oder Schutzfaktoren, insbesondere gewebespezifische Plasminogenaktivatoren, Serumalbumine und/oder CMC. Ebenso kann das Druckmedium beziehungsweise eine daraus erzeugte Zellkultur 22 und/oder Zellagglomeration 24 zumindest eine oder mehrere dieser Substanzen aus den vorstehend benannten Gruppen aufweisen.

Die gemäß voranstehend beschriebenen Verfahren erzeugten Zellenkulturen 22 und/oder Zellagglomerationen 24 eignen sich besonders bevorzugt zur Durchführung von medizinischen und/oder pharmakologischen Untersuchungen.

## Patentansprüche

1. Verfahren zur Erzeugung und/oder Anordnung von Zellkulturen (22) und/oder Zellagglomerationen (24), insbesondere für die Durchführung medizinischer und/oder pharmakologischer Untersuchungen, bei dem ein lebende Zellen enthaltenes Druckmedium bereitgestellt wird und bei dem das Druckmedium durch ein Drucksieb und/oder eine Druckschablone (14) hindurch gedruckt wird.

2. Verfahren nach Anspruch 1, **dadurch gekennzeichnet, dass** das Druckmedium durch ein Drucksieb und/oder eine Druckschablone (14) auf eine Trägerstruktur (10) gedruckt wird und/oder dass eine Trägerstruktur (10) für eine Mehrzahl voneinander getrennter Zellkulturen (22) und/oder Zellagglomerationen (24) bereitgestellt wird und/oder dass eine Trägerstruktur (10) zusammen und/oder abwechselnd mit dem lebende Zellen enthalten-den Druckmedium gedruckt wird und/oder dass eine Trägerstruktur (10) vor dem Drucken des lebende Zellen enthaltenes Druckmediums gedruckt wird.

3. Verfahren nach Anspruch 1 oder 2, **dadurch gekennzeichnet, dass** das Druckmedium durch mindestens einen Rakelvorgang und/oder mindestens eine Druckrakelbewegung, insbesondere durch eine Mehrzahl von Rakelvorgängen und/oder eine Mehrzahl von Druckrakelbewegungen, durch das Drucksieb und/oder die Druckschablone (14) hindurch und/oder auf eine Trägerstruktur (10) befördert wird.

4. Verfahren nach einem der vorstehenden Ansprüche, **dadurch gekennzeichnet, dass** das Druckmedium durch mindestens einen Siebdruckvorgang, insbesondere durch eine Mehrzahl von Siebdruckvorgängen, gedruckt wird, insbesondere auf eine Trägerstruktur (10), und/oder dass das Drucken des Druckmediums im Siebdruckverfahren erfolgt, insbesondere im 2D-Siebdruckverfahren oder 3D-Siebdruckverfahren, und/oder dass das Drucken des lebende Zellen enthaltenden Druckmediums zusammen und/oder abwechselnd mit dem Drucken der Trägerstruktur (10) im Siebdruckverfahren erfolgt und/oder dass durch den Druck des Druckmediums ein- oder mehrschichtige Zellkulturen (22) und/oder ein- oder mehrschichtige Zellagglomerationen (24) erzeugt werden.

5. Verfahren nach einem der vorstehenden Ansprüche, **dadurch gekennzeichnet, dass** durch das Bedrucken einer Trägerstruktur (10) mit dem Druckmedium eine dreidimensionale Zellkultur (22) und/oder Zellagglomeration (24) erzeugt wird und/oder dass sich nach dem Bedrucken einer Trägerstruktur (10) mit dem Druckmedium die jeweilige Zellkultur (22) und/oder Zellagglomeration (24) zu einer dreidimensionalen Zellkultur (22) und/oder Zellagglomeration (24) entwickelt und/oder dass in Druckabschnitten (26) strukturelle Gerüste und/oder Scaffolds vorgesehen sind und/oder dass strukturelle Gerüste und/oder Scaffolds gemeinsam mit dem Druckmedium gedruckt werden und/oder in diesem enthalten sind und/oder dass sich die Zellkulturen (22) und/oder die Zellagglomerationen (24) nach dem Drucken zu Gewebe entwickeln.

6. Verfahren nach einem der Ansprüche 2 bis 5, **dadurch gekennzeichnet, dass** die Trägerstruktur (10) für die Anordnung und/oder Aufnahme einer Mehrzahl voneinander getrennter Zellkulturen (22) und/oder Zellagglomeration (24) ausgebildet ist und/oder dass die Trägerstruktur (10) vordefinierte Anordnungs- und/oder Aufnahmeabschnitte (12) für voneinander getrennte Zellkulturen (22) und/oder Zellagglomeration (24) aufweist und/oder dass die Anordnungs- und/oder Aufnahmeabschnitte (12) in mehreren Zeilen und Reihen auf der Trägerstruktur (10) vorgesehen sind und/oder dass die Trägerstruktur (10) als Objektträger und/oder Zellkulturplatte ausgebildet ist und/oder Vertiefungen zur Aufnahme des Druckmediums aufweist.

7. Verfahren nach einem der vorstehenden Ansprüche, **dadurch gekennzeichnet, dass** das Drucksieb und/oder die Druckschablone (14) eine Mehrzahl von Ausschnitten (18) für den Durchlass des Druckmediums aufweist, insbesondere getrennt voneinander ausgebildete Ausschnitte (18), wobei die Ausschnitte (18) des Drucksiebs und/oder der Druckschablone (14) bevorzugt mit vordefinierten Anordnungs- und/oder Aufnahmeabschnitten (12) der Trägerstruktur (10) korrespondieren.

8. Verfahren nach einem der vorstehenden Ansprüche, **dadurch gekennzeichnet, dass** zumindest ein Ausschnitt (18) des Drucksiebs und/oder der Druckschablone (14) für den Durchlass des Druckmediums eine Größe und/oder einen Durchmesser von 1 mm bis 100 mm, insbesondere von 2 mm bis 50 mm, bevorzugt von 2 mm bis 40 mm, bevorzugt von 2 mm bis 30 mm, weiter bevorzugt von 3 mm bis 30 mm, weiter bevorzugt von 3 mm bis 25 mm, weiter bevorzugt von 3 mm bis 20 mm, noch weiter bevorzugt von 4 mm bis 20 mm, weiter bevorzugt von 4 mm bis 15 mm, weiter bevorzugt von 4 mm bis 10 mm, noch weiter bevorzugt von 4 mm bis 9 mm, weiter bevorzugt von 4 mm bis 8 mm, noch weiter bevorzugt von 5 mm bis 10 mm, weiter bevorzugt von 5 mm bis 8 mm, aufweist.

9. Verfahren nach einem der Ansprüche 2 bis 8, **dadurch gekennzeichnet, dass** eine Mehrzahl von Anordnungs- und/oder Aufnahmeabschnitten (12) der Trägerstruktur (10) zeitgleich und/oder in einem Druckvorgang und/oder durch einen Rakelvorgang mit dem Druckmedium bedruckt und/oder befüllt werden und/oder dass das Druckmedium an vereinzelten Positionen auf eine Trägerstruktur (10) gedruckt wird und/oder dass durch das Bedrucken einer Trägerstruktur (10) mit dem Druckmedium voneinander unabhängige und/oder fluidtechnisch getrennte Druckabschnitte (26) erzeugt werden.

10. Verfahren nach einem der vorstehenden Ansprüche, **dadurch gekennzeichnet, dass** während des Druckens, vor dem Drucken und/oder nach dem Drucken eine Überlagerung des Druckmediums mit einem sterilem Gasmedium erfolgt, insbesondere mit steriler Luft.

11. Verfahren nach einem der vorstehenden Ansprüche, **dadurch gekennzeichnet, dass** das Druckmedium als Druckpaste und/oder niedrigviskose oder mittelviskose Nährflüssigkeit und/oder flüssige Suspension und/oder Sol-Gel Matrix ausgebildet ist und/oder dass das Druckmedium eine variable Viskosität aufweist und/oder dass die Viskosität des Druckmediums durch einen Trocknungsschritt und/oder Temperierungsschritt veränderbar ist und/oder dass das Druckmedium strukturviskos ist.

12. Verfahren nach einem der vorstehenden Ansprüche, **dadurch gekennzeichnet, dass** zwischen aufeinanderfolgenden Druckschritten ein Trocknungs- und/oder Temperierungsschritt des aufgedruckten Druckmediums erfolgt und/oder dass nach dem Drucken des Druckmediums ein Sol-Gel-Übergang erzeugt wird, insbesondere durch einen Trocknungs- und/oder Temperierungsschritt, und/oder dass nach einem erzeugten Sol-Gel-Übergang ein weiterer Druckvorgang vorgenommen wird.

13. Verfahren zur Erzeugung und/oder Anordnung von Zellkulturen (22) und/oder Zellagglomerationen (24), insbesondere nach einem der vorstehenden Ansprüche, bei dem eine Trägerstruktur (10) für eine Mehrzahl voneinander getrennter Zellkulturen (22) und/oder Zellagglomerationen (24) bereitgestellt und ein lebende Zellen enthaltenes Druckmedium mittels Siebdrucks auf die Trägerstruktur (10) gedruckt wird.

## Claims

1. Method for producing and/or arranging cell cultures (22) and/or cell agglomerations (24), in particular for carrying out medical and/or pharmacological examinations, in which a printing medium containing living cells is provided and in which the printing medium is printed through a printing screen and/or a printing stencil (14).

2. Method according to claim 1, **characterized in that** the printing medium is printed through a printing screen and/or a printing stencil (14) onto a carrier structure (10) and/or that a carrier structure (10) is provided for a plurality of separate cell cultures (22) and/or cell agglomerations (24) and/or that a carrier structure (10) is printed together with and/or alternately with the printing medium containing living cells and/or that a carrier structure (10) is printed before the printing medium containing living cells is printed.

3. Method according to claim 1 or 2, **characterized in that** the printing medium is transported through the printing screen and/or the printing stencil (14) and/or onto a carrier structure (10) by at least one squeegee operation and/or at least one printing squeegee movement, in particular by a plurality of squeegee operations and/or a plurality of printing squeegee movements.

4. Method according to one of the preceding claims, **characterized in that** the printing medium is printed by at least one screen printing operation, in particular by a plurality of screen printing operations, in particular onto a carrier structure (10), and/or that the printing of the printing medium is carried out using the screen printing process, in particular the 2D screen printing process or 3D screen printing process, and/or that the printing of the printing medium containing living cells is carried out together and/or alternately with the printing of the carrier structure (10) using the screen printing process and/or that single-layer or multi-layer cell cultures (22) and/or single-layer or multi-layer cell agglomerations (24) are produced by printing the printing medium.

5. Method according to one of the preceding claims, **characterized in that** by printing the printing medium onto a carrier structure (10) a three-dimensional cell culture (22) and/or cell agglomeration (24) is produced and/or that after printing the printing medium onto a carrier structure (10), the respective cell culture (22) and/or cell agglomeration (24) develops into a three-dimensional cell culture (22) and/or cell agglomeration (24) and/or that structural scaffolds and/or scaffolds are provided in printing sections (26) and/or that structural scaffolds and/or scaffolds are printed together with the printing medium and/or are contained therein and/or that the cell cultures (22) and/or the cell agglomerations (24) develop into tissue after printing.

6. Method according to one of claims 2 to 5, **characterized in that** the carrier structure (10) is designed for the arrangement and/or reception of a plurality of separate cell cultures (22) and/or cell agglomerations (24) and/or that the carrier structure (10) has predefined arrangement and/or reception sections (12) for separate cell cultures (22) and/or cell agglomerations (24) and/or that the arrangement and/or receiving sections (12) are provided in several rows and columns on the carrier structure (10) and/or that the carrier structure (10) is designed as a microscope slide and/or cell culture plate and/or has recesses for receiving the printing medium.

7. Method according to one of the preceding claims, **characterized in that** the printing screen and/or the printing stencil (14) has a plurality of cut-outs (18) for the passage of the printing medium, in particular cut-outs (18) formed separately from one another, wherein the cut-outs (18) of the printing screen and/or the printing stencil (14) preferably correspond to predefined arrangement and/or receiving sections (12) of the carrier structure (10).

8. Method according to one of the preceding claims, **characterized in that** at least one cut-out (18) of the printing screen and/or the printing stencil (14) for the passage of the printing medium has a size and/or a diameter of 1 mm to 100 mm, in particular from 2 mm to 50 mm, preferably from 2 mm to 40 mm, preferably from 2 mm to 30 mm, more preferably from 3 mm to 30 mm, more preferably from 3 mm to 25 mm, more preferably from 3 mm to 20 mm, even more preferably from 4 mm to 20 mm, more preferably from 4 mm to 15 mm, more preferably from 4 mm to 10 mm, even more preferably from 4 mm to 9 mm, more preferably from 4 mm to 8 mm, even more preferably from 5 mm to 10 mm, more preferably from 5 mm to 8 mm.

9. Method according to one of claims 2 to 8, **characterized in that** a plurality of arrangement and/or receiving sections (12) of the carrier structure (10) are printed and/or filled with the printing medium simultaneously and/or in a printing process and/or by a squeegee process, and/or that the printing medium is printed onto a carrier structure (10) at individual positions and/or that printing a carrier structure (10) with the printing medium produces printing sections (26) that are independent of one another and/or separated in terms of fluid technology.

10. Method according to one of the preceding claims, **characterized in that** during printing, before printing and/or after printing, the printing medium is overlaid with a sterile gas medium, in particular with sterile air.

11. Method according to one of the preceding claims, **characterized in that** the printing medium is formed as a printing paste and/or low-viscosity or medium-viscosity nutrient fluid and/or liquid suspension and/or solgel matrix and/or that the printing medium has a variable viscosity and/or that the viscosity of the printing medium can be changed by a drying step and/or temperature control step and/or that the printing medium is structurally viscous.

12. Method according to one of the preceding claims, **characterized in that** a drying and/or temperature control step of the printed printing medium takes place between successive printing steps and/or that a sol-gel transition is produced after printing the printing medium, in particular by a drying and/or temperature control step, and/or that a further printing process is carried out after a sol-gel transition has been produced.

13. Method for producing and/or arranging cell cultures (22) and/or cell agglomerations (24), in particular according to one of the preceding claims, in which a carrier structure (10) is provided for a plurality of separate cell cultures (22) and/or cell agglomerations (24) and a printing medium containing living cells is printed onto the carrier structure (10) by means of screen printing.

## Revendications

1. Procédé pour la production et/ou la disposition de cultures cellulaires (22) et/ou d'agglomérations cellulaires (24), en particulier pour la réalisation d'examens médicaux et/ou pharmacologiques, dans lequel un milieu d'impression contenant des cellules vivantes est mis à disposition et dans lequel le milieu d'impression est imprimé à travers un écran d'impression et/ou un patron d'impression (14).

2. Procédé selon la revendication 1, **caractérisé en ce que** le milieu d'impression est imprimé sur une structure de support (10) à travers un écran d'impression et/ou un patron d'impression (14) et/ou **en ce qu'**une structure de support (10) est prévue pour une pluralité de cultures cellulaires (22) et/ou d'agglomérations cellulaires (24) séparées les unes des autres et/ou **en ce qu'**une structure de support (10) est imprimée conjointement et/ou en alternance avec le milieu d'impression contenant des cellules vivantes et/ou **en ce qu'**une structure de support (10) est imprimée avant l'impression du milieu d'impression contenant des cellules vivantes.

3. Procédé selon la revendication 1 ou 2, **caractérisé en ce que** le milieu d'impression est transporté par au moins un processus de raclage et/ou au moins un mouvement de raclage d'impression, en particulier par une pluralité de processus de raclage et/ou une pluralité de mouvements de raclage d'impression, à travers l'écran d'impression et/ou le patron d'impression (14) et/ou sur une structure de support (10).

4. Procédé selon l'une des revendications précédentes, **caractérisé en ce que** le milieu d'impression est imprimé par au moins un processus de sérigraphie, en particulier par une pluralité de processus de sérigraphie, en particulier sur une structure de support (10), et/ou **en ce que** l'impression du milieu d'impression est réalisée lors d'un procédé de sérigraphie, en particulier lors d'un procédé de sérigraphie 2D ou lors d'un procédé de sérigraphie 3D, et/ou **en ce que** l'impression du milieu d'impression contenant des cellules vivantes est effectuée en même temps et/ou en alternance avec l'impression de la structure de support (10) lors du procédé de sérigraphie et/ou **en ce que** des cultures cellulaires (22) à une ou plusieurs couches et/ou des agglomérations cellulaires (24) à une ou plusieurs couches sont produites par l'impression du milieu d'impression.

5. Procédé selon l'une des revendications précédentes, **caractérisé en ce qu'**une culture cellulaire tridimensionnelle (22) et/ou une agglomération cellulaire (24) tridimensionnelle sont produites par l'impression d'une structure de support (10) avec le milieu d'impression et/ou **en ce que**, après l'impression d'une structure de support (10) avec le milieu d'impression, la culture cellulaire (22) et/ou l'agglomération cellulaire (24) respectives se développent en une culture cellulaire tridimensionnelle (22) et/ou en une agglomération cellulaire tridimensionnelle (24) et/ou **en ce que** des ossatures structurelles et/ou des armatures de soutien structurelles sont prévues dans des sections d'impression (26) et/ou **en ce que** des ossatures structurelles et/ou des armatures de soutien structurelles sont imprimées conjointement avec le milieu d'impression et/ou sont contenues dans celui-ci et/ou **en ce que** les cultures cellulaires (22) et/ou les agglomérations cellulaires (24) se développent en tissu après l'impression.

6. Procédé selon l'une des revendications 2 à 5, **caractérisé en ce que** la structure de support (10) est conçue pour la disposition et/ou la réception d'une pluralité de cultures cellulaires (22) et/ou d'agglomération cellulaires (24) séparées les unes des autres et/ou **en ce que** la structure de support (10) présente des sections de disposition et/ou de réception (12) prédéfinies pour des cultures cellulaires (22) et/ou agglomérations cellulaires (24) séparées les unes des autres et/ou **en ce que** les sections de disposition et/ou de réception (12) sont prévues en plusieurs lignes et rangées sur la structure de support (10) et/ou **en ce que** la structure de support (10) est réalisée sous forme de support pour objet et/ou de plaque pour culture cellulaire et/ou présente des renfoncements pour la réception du milieu d'impression.

7. Procédé selon l'une des revendications précédentes, **caractérisé en ce que** l'écran d'impression et/ou le patron d'impression (14) présentent une pluralité de découpes (18) pour le passage du milieu d'impression, en particulier des découpes (18) réalisées séparément les unes des autres, dans lequel les découpes (18) de l'écran d'impression et/ou du patron d'impression (14) correspondent de préférence à des sections de disposition et/ou de réception (12) prédéfinies de la structure de support (10).

8. Procédé selon l'une des revendications précédentes, **caractérisé en ce qu'**au moins une découpe (18) de l'écran d'impression et/ou du patron d'impression (14) pour le passage du milieu d'impression a une taille et/ou un diamètre allant de 1 mm à 100 mm, en particulier de 2 mm à 50 mm, de préférence de 2 mm à 40 mm, de préférence de 2 mm à 30 mm, plus préférablement de 3 mm à 30 mm, plus préférablement de 3 mm à 25 mm, plus préférablement de 3 mm à 20 mm, encore plus préférablement de 4 mm à 20 mm, plus préférablement de 4 mm à 15 mm, plus préférablement de 4 mm à 10 mm, encore plus préférablement de 4 mm à 9 mm, plus préférablement de 4 mm à 8 mm, encore plus préférablement de 5 mm à 10 mm, plus préférablement de 5 mm à 8 mm.

9. Procédé selon l'une des revendications 2 à 8, **caractérisé en ce qu'**une pluralité de sections de disposition et/ou de réception (12) de la structure de support (10) sont imprimées avec le milieu d'impression et/ou remplies de celui-ci simultanément et/ou lors d'un processus d'impression et/ou par un processus de raclage et/ou **en ce que** le milieu d'impression est imprimé sur une structure de support (10) en des positions isolées et/ou **en ce que** des sections d'impression (26) indépendantes les unes des autres et/ou séparées de manière fluidique sont produites par l'impression d'une structure de support (10) avec le milieu d'impression.

10. Procédé selon l'une des revendications précédentes, **caractérisé en ce que**, pendant l'impression, avant l'impression et/ou après l'impression, une superposition du milieu d'impression avec un milieu gazeux stérile, en particulier avec de l'air stérile, est effectuée.

11. Procédé selon l'une des revendications précédentes, **caractérisé en ce que** le milieu d'impression est réalisé sous forme de pâte d'impression et/ou de liquide nutritif de faible viscosité ou de moyenne viscosité et/ou de suspension liquide et/ou de matrice sol-gel et/ou **en ce que** le milieu d'impression présente une viscosité variable et/ou **en ce que** la viscosité du milieu d'impression peut être modifiée par une étape de séchage et/ou une étape de régulation de température et/ou **en ce que** le milieu d'impression est à viscosité intrinsèque.

12. Procédé selon l'une des revendications précédentes, **caractérisé en ce qu'**entre des impressions successives, une étape de séchage et/ou de régulation de température du support d'impression imprimé est effectuée et/ou **en ce qu'**après l'impression du support d'impression, une transition sol-gel est produite, en particulier par une étape de séchage et/ou de régulation de température, et/ou **en ce qu'**après une transition sol-gel produite, un autre processus d'impression est réalisé.

13. Procédé pour la production et/ou la disposition de cultures cellulaires (22) et/ou d'agglomérations cellulaires (24), en particulier selon l'une des revendications précédentes, dans lequel une structure de support (10) est mise à disposition pour une pluralité de cultures cellulaires (22) et/ou d'agglomérations cellulaires (24) séparées les unes des autres et un milieu d'impression contenant des cellules vivantes est imprimé par sérigraphie sur la structure de support (10).
